# EUROPEAN PATENT APPLICATION

(11) **EP 4 819 157 A1**
(43) Date of publication of application: **07.10.2026**
(21) Application number: 24898041.9
(22) Date of filing: 25.11.2024
(51) Int. Cl.: G16H 50/20, G16H 10/60, G16H 10/20, G16H 50/30, G16H 40/20, G16H 70/20, G06Q 50/10, G06V 30/40, G10L 15/02, G08B 27/00

(54) **EMERGENCY MEDICAL SYSTEM AND METHOD FOR VEHICLE FOR GUIDING EMERGENCY PATIENT CLASSIFICATION, TREATMENT, AND HOSPITAL TRANSPORT**

(30) Priority: 28.11.2023 KR 20230167481; 15.11.2024 KR 20240162863
(71) Applicant: Cosmos Medic Inc., Seongnam-si Gyeonggi-do 13449 (KR)
(72) Inventor: KIM, Ji Hun, Yongin-si Gyeonggi-do 16955 (KR)
(74) Representative: Viering, Jentschura & Partner mbB Patent- und Rechtsanwälte
(86) International application number: PCT/KR2024/018706
(87) International publication number: WO 2025/116446

(57) **Abstract**

An emergency medical method for a vehicle for guiding emergency patient classification, treatment, and hospital transport, according to the present invention, comprises the steps in which: a patient information registration unit receives essential information on a patient before boarding a vehicle or inside the vehicle after boarding; a patient symptom input unit receives the patient's current symptoms in various formats; an emergency treatment guideline provision unit presents emergency treatment methods suitable for the patient on the basis of the collected information; and a remote medical support unit provides support for real-time communication with remote medical staff inside the vehicle so that the patient or paramedics can receive direct advice and guidance from an emergency medical specialist.

## Description

### Technical Field

The present disclosure relates to an emergency medical system and method and, more specifically, to an emergency medical system and method for a vehicle for guiding laypersons in performing emergency patient classification, treatment, and transport to hospital, which can improve a survival rate of a patient and a quality of treatment by enabling both ordinary vehicle users and medical personnel to more quickly and effectively assess and manage a condition of the patient in an emergency situation occurring within a vehicle.

### Background Art

In the current medical market, there is a lack of effective solutions for managing emergency situations occurring within a vehicle. Most emergency medical software or systems are primarily developed for medical professionals or emergency responders and are not designed to be easily used by ordinary vehicle users within the vehicle. This makes it difficult for laypersons to appropriately respond to emergency situations (e.g., traffic accidents, sudden health issues, cardiac arrest, etc.) occurring within the vehicle. In addition, even medical personnel may find it practically difficult to be fully acquainted with and proficiently respond to all types of emergency situations, so there is a need for software capable of assisting in emergency treatment.

Conventional emergency medical systems do not sufficiently take into account the unique characteristics of the in-vehicle usage environment. The interior of the vehicle is a confined space, vibrations and noise are generated during movement, and operation during driving is subject to safety constraints. In such an environment, there is a need for a voice-based interface, which enables hands-free operation, or a simplified control method, but existing solutions (e.g., software) fail to meet these requirements.

Despite the advancement of autonomous vehicles, a system that integrates and utilizes sensor data and control systems of autonomous vehicles in conjunction with emergency medical services has not yet been developed. Autonomous vehicles are capable of monitoring vehicle conditions and surrounding environments using various sensors and data, but methods for utilizing such information in responding to emergency situations remain insufficient. This means that potential emergency medical support functions provided by the autonomous vehicle are not being fully utilized.

In addition, insufficient integration between emergency medical devices (e.g., an automated external defibrillator (AED), an oxygen supply device, etc.) mounted within the vehicle and software presents a further problem. Conventional emergency medical systems lack adequate integration with such devices (e.g., an automated external defibrillator (AED), an oxygen supply device, etc.), thereby failing to support effective use of the devices in emergency situations. This results in delays in appropriate treatment or prevents the full utilization of the device's functions, in a case where a user is not sufficiently familiar with operation of the devices.

Meanwhile, although remote medical support technologies have advanced, systems that enable real-time communication with emergency medicine specialists within the vehicle have not yet been widely implemented. Accordingly, in complex or critical emergency situations, it is difficult to promptly obtain professional medical advice, thereby reducing the effectiveness and accuracy of emergency treatment.

There is a lack of user-friendly interfaces and practical first-aid guidelines that enable laypersons to perform appropriate emergency treatment without specialized medical knowledge in emergency situations. Conventional first-aid instructions or educational materials are typically composed of technical terminology and complex procedures, thereby making it difficult for non-professionals to readily understand and apply the information in urgent situations.

In addition, in terms of data security and personal information protection, there is a lack of a framework for securely managing and transmitting sensitive medical information collected within the vehicle. This is one of the factors that discourage users from utilizing emergency medical systems and increases concerns regarding leakage or misuse of medical information.

Korean Patent Application Publication No. 10-2021-0054978 (Patent Document 1) discloses "Treatment Method of Emergency Patient during Transporting by Vehicle," and according thereto, the method for treating an emergency patient during vehicle transfer includes: a first step of detecting an emergency patient lying on an upper surface of a bed inside a vehicle to generate an operation event; a second step of switching an operation of a medical auxiliary module installed within the vehicle to an ON state in response to the operation event; a third step of recognizing a face of the emergency patient through the medical auxiliary module to identify the emergency patient; a fourth step of generating first information on allergy-related drugs highly requiring attention and second information related to emergency medicine, after analyzing a past medical record of the emergency patient through the medical auxiliary module using a deep learning-based artificial intelligence technology; and a fifth step of transmitting the first information and the second information to a server within an emergency institution.

As described above, Patent Document 1 has effects of providing a method for rapidly performing emergency treatment on a critically ill patient being transported by an emergency vehicle, and
of easily identifying the emergency patient on the basis of past data to provide appropriate information to a rescuer in performing emergency treatment, such as drug administration, but does not provide means for securely managing and transmitting sensitive medical information of the patient collected within the vehicle, thereby posing risks of leakage or misuse of medical information.

### Disclosure

### Technical Problem

The present disclosure has been made by comprehensively taking into account the above-mentioned matters, and aims to provide an emergency medical system and method for a vehicle for guiding laypersons in performing emergency patient classification, treatment, and transport to hospital, which is capable of improving a survival rate of the patient and a quality of emergency medical services by responding to various emergency situations occurring within the vehicle, by supporting rapid treatment decision-making through integration between the vehicle and emergency medical devices, and then by enabling both ordinary vehicle users and medical personnel to more quickly and effectively assess and manage a condition of the patient in an emergency situation occurring within the vehicle.

Another objective of the present disclosure is to provide an emergency medical system and method for a vehicle for guiding laypersons in performing emergency patient classification, treatment, and transport to hospital, which is capable of removing factors that discourage users from utilizing emergency medical systems and of alleviating concerns regarding leakage or misuse of medical information by providing means for securely managing and transmitting sensitive medical information of the patient collected within the vehicle.

### Technical Solution

An emergency medical system for a vehicle for guiding laypersons in performing emergency patient classification, treatment, and transport to hospital according to the present disclosure for achieving the above objectives includes
a patient information registration unit for receiving essential information of a patient from the patient or an emergency responder, prior to boarding the vehicle or within the vehicle after boarding;
a patient symptom input unit for receiving current symptoms of the patient in various forms from the patient or the emergency responder;
a data security and personal information management unit configured to encrypt and store the input patient information, to require a user to perform a consent procedure for collection and use of the information, and to allow only authorized users to access the corresponding information;
an emergency treatment guideline provision unit for providing an appropriate emergency treatment method for the patient on the basis of the collected information;
a vehicle and emergency medical device interworking unit configured to interwork with sensor data and a control system of an autonomous vehicle, and to support the vehicle to automatically travel to a nearest medical institution or stop at a safe location when an emergency situation occurs;
a remote medical support unit for supporting real-time communication with remote medical personnel within the vehicle so that the patient or the emergency responder can receive direct advice and guidance from an emergency medicine specialist; and
a control unit configured to control operations and perform state monitoring of the patient information registration unit, the patient symptom input unit, the data security and personal information management unit, the emergency treatment guideline provision unit, the vehicle and emergency medical device interworking unit, and the remote medical support unit, to interwork with an existing medical information platform so as to utilize personal information of the patient, and to share information with the medical institution when necessary in the emergency situation to support rapid medical treatment.

Herein, preferably, an external medical data interworking unit may be further included which interworks with a national medical MyData platform so as to automatically retrieve and utilize medical records, underlying conditions, and allergy information, and the like of the patient.

In addition, the essential information received by the patient information registration unit may include at least one of a name, an age, a sex, contact information, a height, a weight, an underlying condition, medication information, and surgical history of the patient.

In addition, the patient symptom input unit may receive current symptoms of the patient in at least one form selected from the group consisting of text, an image, audio, a video, and biometric data.

In addition, the emergency treatment guideline provision unit may provide the appropriate emergency treatment method for the patient using an emergency treatment guideline generated on the basis of emergency medicine textbooks, latest papers and guidelines, and clinical experience of medical personnel, or a pre-trained artificial intelligence (AI)-based emergency treatment model.

In addition, the vehicle and emergency medical device interworking unit may monitor, in real time, changes in a condition of the patient using an in-vehicle sensor, and automatically contact an emergency medical service center when necessary.

In addition, in a case of a non-autonomous vehicle, the vehicle and emergency medical device interworking unit may provide optimal route guidance and emergency treatment guidance to a driver via real-time voice guidance and visual alerts.

In addition, the vehicle and emergency medical device interworking unit may be configured to interwork with an emergency medical device provided within the vehicle, including an automated external defibrillator (AED), an oxygen supply device, and a heart rate monitor, to monitor a biometric signal of the patient in real time and to automatically operate the emergency medical device when necessary.

In addition, the remote medical support unit may support receipt of the advice and guidance via at least one of a video call, a voice call, and chat.

In addition, the control unit may interwork with the existing medical information platform to utilize the personal information of the patient, and the personal information of the patient may include at least one selected from the group consisting of medical records, underlying conditions, and allergy information of the patient.

In addition, an emergency medical method for a vehicle for guiding laypersons in performing emergency patient classification, treatment, and transport to hospital according to the present disclosure for achieving the above objectives includes,
a) receiving, by a patient information registration unit, essential information of a patient from the patient or an emergency responder, prior to boarding the vehicle or within the vehicle after boarding;
b) receiving, by a patient symptom input unit, current symptoms of the patient in various forms from the patient or the emergency responder;
c) providing, by an emergency treatment guideline provision unit, an appropriate emergency treatment method for the patient on the basis of the collected information;
d) interworking, by a vehicle and emergency medical device interworking unit, with sensor data and a control system of an autonomous vehicle, and supporting the vehicle to automatically travel to a nearest medical institution or stop at a safe location when an emergency situation occurs;
e) supporting, by a remote medical support unit, real-time communication with remote medical personnel within the vehicle so that the patient or the emergency responder can receive direct advice and guidance from an emergency medicine specialist;
f) interworking, by a control unit, with an existing medical information platform so as to utilize personal information of the patient; and
g) sharing, by the control unit, information with the medical institution when necessary in the emergency situation to support rapid medical treatment.

Herein, in step (a), the essential information received by the patient information registration unit may include at least of a name, an age, a sex, contact information, a height, a weight, an underlying condition, medication information, and surgical history of the patient.

In addition, in step (b), the patient symptom input unit may receive current symptoms of the patient in at least one form selected from the group consisting of text, an image, audio, a video, and biometric data.

In addition, in step (c), the emergency treatment guideline provision unit may provide an appropriate emergency treatment method for the patient using an emergency treatment guideline generated on the basis of emergency medicine textbooks, latest papers, guidelines, and clinical experience of medical personnel, or a pre-trained artificial intelligence (AI)-based emergency treatment model.

In addition, in step (d), the vehicle and emergency medical device interworking unit may monitor, in real time, changes in a condition of the patient using an in-vehicle sensor, and automatically contact an emergency medical service center when necessary.

In addition, in step (d), in a case of a non-autonomous vehicle, the vehicle and emergency medical device interworking unit may provide optimal route guidance and emergency treatment guidance to a driver via real-time voice guidance and visual alerts.

In addition, in step (d), the vehicle and emergency medical device interworking unit may be configured to interwork with an emergency medical device provided within the vehicle, including an automated external defibrillator (AED), an oxygen supply device, and a heart rate monitor, to monitor a biometric signal of the patient in real time and to automatically operate the emergency medical device when necessary.

In addition, in step (e), the remote medical support unit may support receipt of the advice and guidance via at least one of a video call, a voice call, and chat.

In addition, in step (f), the personal information of the patient may include at least one selected from the group consisting of medical records, underlying conditions, and allergy information of the patient.

### Advantageous Effects

According to the present disclosure as described above, it is possible to respond to various emergency situations occurring within a vehicle and to support rapid treatment decision-making through integration between the vehicle and emergency medical devices, such that both ordinary vehicle users and medical personnel can quickly and effectively assess and manage a condition of a patient in an emergency situation occurring within the vehicle, thereby improving a survival rate of the patient and a quality of emergency medical services.

In addition, there are effects of removing factors that discourage users from utilizing emergency medical systems and of alleviating concerns regarding leakage or misuse of medical information by providing means for securely managing and transmitting sensitive medical information of the patient collected within the vehicle.

### Description of Drawings

FIG. 1 is a view schematically illustrating a configuration of an emergency medical system for a vehicle for guiding laypersons in performing emergency patient classification, treatment, and transport to hospital according to the present disclosure.
FIG. 2 is a flowchart illustrating an execution process of an emergency medical method for a vehicle for guiding laypersons in performing emergency patient classification, treatment, and transport to hospital according to the present disclosure.

### Mode for Invention

Hereinafter, exemplary embodiments of the present disclosure will be described in detail with reference to the accompanying drawings.

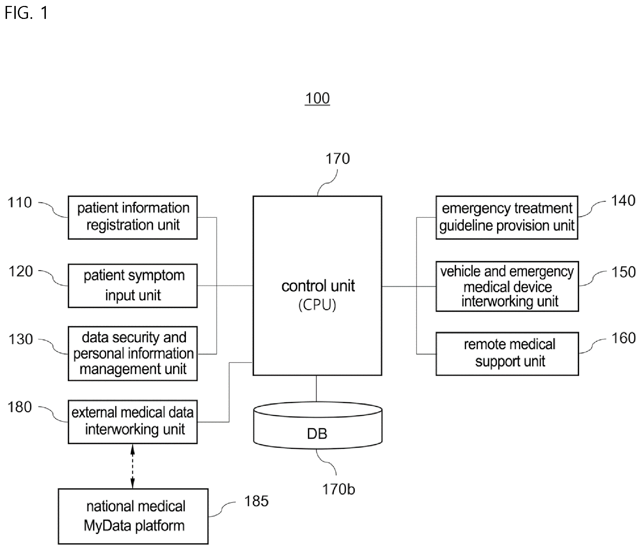

FIG. 1 is a view schematically illustrating a configuration of an emergency medical system for a vehicle for guiding laypersons in performing emergency patient classification, treatment, and transport to hospital according to the present disclosure.

Referring to FIG. 1, an emergency medical system 100 for a vehicle for guiding laypersons in performing emergency patient classification, treatment, and transport to hospital according to the present disclosure may include a patient information registration unit 110, a patient symptom input unit 120, a data security and personal information management unit 130, an emergency treatment guideline provision unit 140, a vehicle and emergency medical device interworking unit 150, a remote medical support unit 160, and a control unit 170.

The patient information registration unit 110 may receive essential information of a patient from the patient or an emergency responder, prior to boarding a vehicle or within the vehicle after boarding. Herein, the essential information received by the patient information registration unit 110 may include at least one of a name, an age, a sex, contact information, a height, a weight, an underlying condition, medication information, and surgical history of the patient.

The patient symptom input unit 120 may receive current symptoms of a patient from the patient or an emergency responder in various forms. The patient symptom input unit 120 may receive the current symptoms of the patient in at least one form of text, an image, audio, a video, and biometric data.

The data security and personal information management unit 130 may encrypt and store the input information of the patient, allow the user to perform a consent procedure for collecting and utilizing information, and allow only the authorized user to access the corresponding information.

The emergency treatment guideline provision unit 140 may provide an appropriate emergency treatment method for a patient on the basis of collected information. Herein, the emergency treatment guideline provision unit 140 may provide the appropriate emergency treatment method using an emergency treatment guideline generated on the basis of emergency medicine textbooks, latest papers and guidelines, and clinical experience of medical personnel, or using a pre-trained artificial intelligence (AI)-based emergency treatment model.

The vehicle and emergency medical device interworking unit 150 may interwork with sensor data and a control system of an autonomous vehicle to support the vehicle to automatically travel to a nearest medical institution or stop at a safe location when an emergency situation occurs. The vehicle and emergency medical device interlocking unit 150 may be provided with a function to monitor changes in a condition of a patient in real time by using in-vehicle sensors, and to automatically contact the emergency medical service center when necessary. In addition, in the case of the non-autonomous vehicle, the vehicle and the emergency medical device interlocking unit 150 may provide optimal route guidance and emergency treatment guidance to a driver through real-time voice guidance and visual alerts. In addition, the vehicle and emergency medical device interworking unit 150 may interwork with emergency medical devices provided within the vehicle, including an automated external defibrillator (AED), an oxygen supply device, and a heart rate monitor, to monitor biometric signals of the patient in real time and to automatically operate the emergency medical devices when necessary.

The remote medical support unit 160 may support real-time communication, within the vehicle, with remote medical personnel so that a patient or an emergency responder can receive direct advice and guidance from an emergency medicine specialist. The remote medical support unit 160 may support the receipt of the advice and guidance via at least one of a video call, a voice call, and chat.

The control unit 170 may be configured to check conditions and control operations of each of the patient information registration unit 110, the patient symptom input unit 120, the data security and personal information management unit 130, the emergency treatment guideline provision unit 140, the vehicle and emergency medical device interworking unit 150, and the remote medical support unit 160, to interwork with an existing medical information platform to utilize personal information of the patient, and to support rapid medical treatment by sharing information with a medical institution when necessary in an emergency situation. Herein, in enabling the control unit 170 to utilize the patient's personal information by interworking with the existing medical information platform, the personal information of the patient may include at least one of medical records, underlying conditions, and allergy information of the patient. In FIG. 1, reference numeral 170b may represent a database in which an OS and various applications required for system operation, as well as various information and data related to emergency patient classification and treatment and hospital transport, are stored.

Herein, the emergency medical system 100 having the above-described configuration for a vehicle for guiding laypersons in performing emergency patient classification, treatment, and transport to hospital according to the present disclosure may, preferably, further include an external medical data interworking unit 180 for interworking with a national medical MyData platform 185 to automatically retrieve and utilize medical records, underlying conditions, and allergy information of the patient.

In addition, the patient information registration unit 110, the patient symptom input unit 120, the data security and personal information management unit 130, the emergency treatment guideline provision unit 140, the vehicle and emergency medical device interworking unit 150, the remote medical support unit 160, and the control unit 170 may be integrated as a whole and configured as one computer system. In this case, the external medical data interworking unit 180 and the database 170b may be integrated together in the above-described computer system or may not be integrated.

Then, hereinafter, an emergency medical method for a vehicle, based on the emergency medical system having the above-described configuration for a vehicle for guiding laypersons in performing emergency patient classification, treatment, and transport to hospital according to the present disclosure will be described.

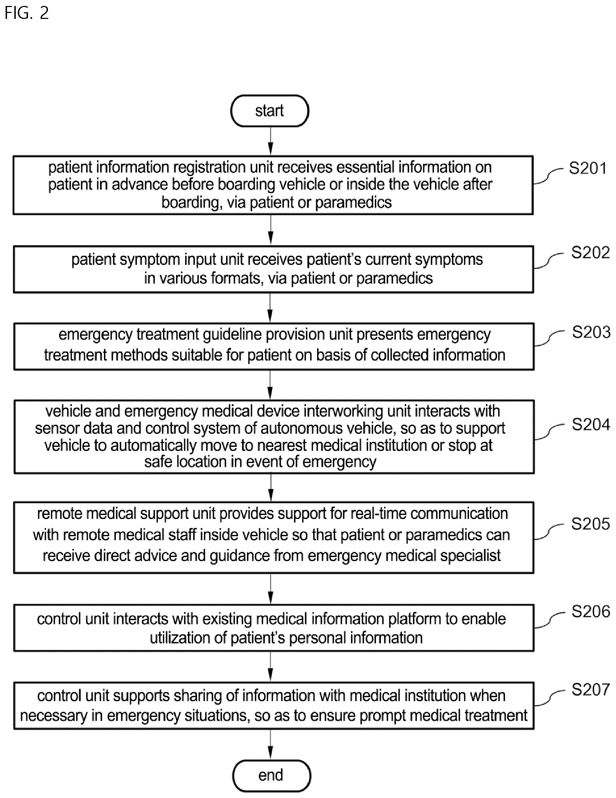

FIG. 2 is a flowchart illustrating an execution process of an emergency medical method for a vehicle for guiding laypersons in performing emergency patient classification, treatment, and transport to hospital according to the present disclosure.

Referring to FIG. 2, in the emergency medical method for a vehicle for guiding laypersons in performing emergency patient classification, treatment, and transport to hospital according to the present disclosure, first, the patient information registration unit 110 may receive essential information of a patient from the patient or an emergency responder, prior to boarding a vehicle or within the vehicle after boarding (step S201). Herein, the essential information received by the patient information registration unit 110 may include at least one of a name, an age, a sex, contact information, a height, a weight, an underlying condition, medication information, and surgical history of the patient.

In addition, the patient symptom input unit 120 may receive current symptoms of the patient from the patient or the emergency responder in various forms (step S202). Herein, the patient symptom input unit 120 may receive the current symptoms in at least one form of text, an image, audio, a video, and biometric data.

In this way, upon receiving the essential information of the patient and the current symptoms of the patient, the emergency treatment guideline provision unit 140 may provide an appropriate emergency treatment method for the patient on the basis of the collected information (step S203). Herein, the emergency treatment guideline provision unit 140 may provide the appropriate emergency treatment method for the patient using an emergency treatment guideline generated on the basis of emergency medicine textbooks, latest papers and guidelines, and clinical experience of medical personnel, or using a pre-trained artificial intelligence (AI)-based emergency treatment model.

Meanwhile, the vehicle and emergency medical device interworking unit 150 may interwork with sensor data and a control system of an autonomous vehicle to support the vehicle to automatically travel to a nearest medical institution or stop at a safe location when an emergency situation occurs (step S204). Herein, the vehicle and emergency medical device interworking unit 150 may monitor changes in a condition of the patient using in-vehicle sensors in real time, and may be provided with a function to automatically contact the emergency medical service center when necessary. In addition, in a case of a non-autonomous vehicle, the vehicle and emergency medical device interworking unit 150 may provide optimal route guidance and emergency treatment guidance to a driver via real-time voice guidance and visual alerts. In addition, the vehicle and emergency medical device interworking unit 150 may interwork with emergency medical devices provided within the vehicle, including an automated external defibrillator (AED), an oxygen supply device, and a heart rate monitor, to monitor biometric signals of the patient in real time and to automatically operate the emergency medical devices when necessary.

In addition, the remote medical support unit 160 may support real-time communication with remote medical personnel within the vehicle so that a patient or an emergency responder can receive direct advice and guidance from an emergency medicine specialist (step S205). Herein, the remote medical support unit 160 may receive the advice and guidance via at least one of a video call, a voice call, and chat.

In the above-described process, the control unit 170 may interwork with an existing medical information platform (e.g., a national medical MyData platform 185) to utilize personal information of the patient (step S206). Herein, the personal information of the patient may include at least one of medical records, underlying conditions, and allergy information of the patient.

In addition, the control unit 170 may share information with a medical institution when necessary in an emergency situation to perform rapid medical treatment (step S207).

Hereinafter, additional description will be provided with respect to the patient information registration unit 110, the patient symptom input unit 120, and the emergency treatment guideline provision unit 140 of an emergency medical system 100 for a vehicle for guiding laypersons in performing emergency patient classification, treatment, and transport to hospital according to the present disclosure described above.

First, additional description will be provided with respect to the patient information registration unit 110.

The patient information registration unit 110 may support various data input methods. That is, information may be received in advance via a web application and a mobile application. A user may input basic information (e.g., a name, an age, a sex, contact information, underlying conditions, medication information, etc.) of a patient in advance through the web application or the mobile application, either prior to boarding a vehicle or after boarding. This may enable information required in an emergency situation to be rapidly utilized.

In addition, the patient information registration unit 110 may utilize scan and OCR technology. That is, identification cards, driver's licenses, passports, medical records, etc., may be scanned within the vehicle, and data may be automatically extracted and entered into the system using OCR (Optical Character Recognition) technology. This may reduce time consumption and errors associated with manual input in conventional techniques.

In addition, the patient information registration unit 110 may include a voice recognition function. That is, even when a user is driving or unable to use hands, the user may input patient information by voice using a high-performance voice recognition function.

In addition, the patient information registration unit 110 may automatically collect biometric data. That is, through interworking with biometric measurement devices (e.g., a heart rate monitor, a blood pressure gauge, etc.) provided within the vehicle, biometric data of the patient may be automatically collected and input into the system.

In addition, the patient information registration unit 110 may provide a user-friendly interface. That is, by designing an intuitive UI/UX, a simplified operation method and an intuitive interface may be provided in consideration of the limited space inside the vehicle and the environment while moving. The interface may include large icons, a simple menu structure, and minimal touch input, thereby enabling even non-professionals to easily use the system.

In addition, the patient information registration unit 110 may support multiple languages, thereby enabling use by foreign users, and may include accessibility features (e.g., voice guidance and screen reader compatibility) for visually and hearing-impaired users. Also, a simple tutorial or help function on how to use the system may be provided so that first-time users can easily adapt to the system.

In addition, the patient information registration unit 110 may update and synchronize data in real time. That is, the input patient information may be securely stored in a cloud server, and the data may be synchronized in real time across multiple devices. This may enable consistency of the information and may allow the latest information to be utilized anytime and anywhere. In addition, an offline mode may be supported to input and store information even in a situation where Internet connection is unstable, and data may be automatically synchronized when the connection is recovered.

In addition, the patient information registration unit 110 may have a function of interworking with Bluetooth and a vehicle system. That is, the voice command input and feedback may be provided through the audio system of the vehicle by interworking with Bluetooth of the vehicle. Also, patient information may be automatically collected and updated by interworking with biometric measurement sensors or a driver monitoring system embedded in the vehicle. In addition, a hands-free operation may be supported so that a system can be safely used during driving, and accordingly, a system can be controlled by a steering wheel button or a voice command.

Next, additional description will be provided with respect to the patient symptom input unit 120.

A simplified symptom input process may be applied to the patient symptom input unit 120. That is, an intuitive interface may be provided on the basis of a predefined symptom list, a medical record of the user, and a symptom frequently occurring, such that the user can quickly input the symptom. This may allow necessary information to be input with minimal operation, thereby minimizing driver distraction during driving.

In addition, the patient symptom input unit 120 may be configured to capture images or videos of a symptom site using an in-vehicle camera and to input the captured images or videos into the system. This can help accurately convey visual symptoms such as wounds, swelling, and rashes. Also, a voice input function may be supported so that the loudness, speed, and intonation of a voice different from usual can be analyzed to recognize an emergency situation.

In addition, by interworking with biometric measurement devices mounted in the vehicle, the patient symptom input unit 120 may collect, in real time, biometric data of a patient, including a heart rate, blood pressure, and oxygen saturation, and monitor the biometric data of the patient together with vehicle sensor data, thereby automatically detecting emergency situations. In addition, when an emergency situation occurs, a notification may be automatically transmitted to an emergency rescue service or remote medical personnel, thereby enabling rapid assistance.

Next, additional description will be provided with respect to the emergency treatment guideline provision unit 140.

The emergency treatment guideline provision unit 140 may automatically evaluate a patient and classify a severity level on the basis of artificial intelligence (AI). That is, an artificial intelligence (AI)-based analysis model trained in advance may analyze input symptoms, biometric data, age, underlying conditions, and the like to evaluate a condition of the patient and automatically classify the severity level. Also, specific symptoms or risk factors may be detected to determine whether urgent medical intervention is required and to determine a priority of actions accordingly.

In addition, the emergency treatment guideline provision unit 140 may provide personalized emergency treatment guidance. That is, the personalized emergency treatment guidance may be provided on the basis of evaluation results of a patient, and the guidance may be presented in detail in a step-by-step manner and may use simplified language and intuitive explanations so that even non-professionals can easily follow the guidance. Also, an emergency treatment method may be visually provided through illustrations, videos, and animations, thereby improving user comprehension.

In addition, the emergency treatment guideline provision unit 140 may support the integration with in-vehicle emergency medical devices and the automated emergency treatment. That is, the patient's biometric signal may be monitored in real time by interworking the installed app (software) with an automated external defibrillator (AED), an oxygen supply device, and a heart rate monitor, etc. installed in the vehicle. Also, depending on the patient's condition, the app may automatically control the appropriate emergency medical device to perform emergency treatment. For example, in a case of a cardiac arrest patient, the app may automatically guide use of the automated external defibrillator and may operate the device when necessary. In addition, step-by-step instructions and voice guidance may be provided so that non-professionals can use the device correctly. This will improve accuracy and effectiveness of emergency treatment.

In addition, the emergency treatment guideline provision unit 140 may have a function of recommending an optimal medical institution and guiding a route thereto. That is, the most appropriate medical institution may be recommended in consideration of a current location, a condition, and required medical services of a patient, and information on specialty, availability of treatment, and facility equipment may be provided by interworking with a hospital database. In addition, an optimal travel route and an estimated arrival time may be provided in consideration of traffic conditions and distance by reflecting real-time traffic information.

In addition, the emergency treatment guideline provision unit 140 may support real-time consultation with remote medical personnel. That is, a function may be provided which connects with an emergency medicine specialist or other medical personnel for real-time consultation. Also, a communication method suitable for a situation may be selected by supporting various communication methods, including video calls, voice calls, and chat. In addition, patient condition information and data may be safely shared with remote medical personnel, thereby receiving accurate diagnosis and advice.

In addition, the emergency treatment guideline provision unit 140 may provide an emergency treatment performance monitoring and feedback function. That is, an emergency treatment performance process may be monitored through the vehicle's sensor data or user input, and immediate feedback and corrective instructions may be provided when the emergency treatment is not properly performed. Also, after the emergency treatment, a change in a condition of the patient may be evaluated to determine effectiveness of the treatment.

As described above, an emergency medical system and method for a vehicle for guiding laypersons in performing emergency patient classification, treatment, and transport to hospital according to the present disclosure has effects of improving a survival rate of the patient and a quality of emergency medical services by responding to various emergency situations occurring within the vehicle, by supporting rapid treatment decision-making through the integration between the vehicle and emergency medical devices, and by enabling both ordinary vehicle users and medical personnel to quickly and effectively assess and manage a condition of a patient in an emergency situation occurring within the vehicle.

In addition, there are effects of removing factors that discourage users from utilizing emergency medical systems, and of alleviating concerns regarding leakage or misuse of medical information by providing means for securely managing and transmitting sensitive medical information of a patient collected within a vehicle.

In addition, from a user (a driver and a passenger) perspective, there are advantages in allowing symptoms to be input by voice without using hands during driving through a voice recognition function by supporting a driver and a passenger to rapidly and effectively respond to emergency situations occurring within a vehicle and in enabling a user to immediately request assistance and perform necessary emergency treatment in various emergency situations occurring on a road, such as traffic accidents or sudden health problems by allowing a pre-trained artificial intelligence (AI)-based model to evaluate a condition of a patient in real time and to determine whether urgent treatment is required.

In addition, from a medical personnel perspective, there are advantages in allowing, by receiving real-time information on the patient's condition and location in the event of an emergency, a hospital to prepare in advance, in identifying a condition of the patient beforehand to appropriately allocate necessary medical equipment and personnel and to establish an effective treatment plan, in minimizing, by receiving detailed information on the patient's past medical records and current condition to enable accurate diagnosis and prompt treatment, delays in an emergency patient treatment process, and in improving work efficiency of medical personnel.

## Claims

1. An emergency medical system for a vehicle for guiding laypersons in performing emergency patient classification, treatment, and transport to hospital, the system comprising:
a patient information registration unit for receiving essential information of a patient from the patient or an emergency responder, prior to boarding the vehicle or within the vehicle after boarding;
a patient symptom input unit for receiving current symptoms of the patient in various forms from the patient or the emergency responder;
a data security and personal information management unit configured to encrypt and store the input patient information, to require a user to perform a consent procedure for collection and use of the information, and to allow only authorized users to access the corresponding information;
an emergency treatment guideline provision unit for providing an appropriate emergency treatment method for the patient on the basis of the collected information;
a vehicle and emergency medical device interworking unit configured to interwork with sensor data and a control system of an autonomous vehicle, and to support the vehicle to automatically travel to a nearest medical institution or stop at a safe location when an emergency situation occurs;
a remote medical support unit for supporting real-time communication with remote medical personnel within the vehicle so that the patient or the emergency responder can receive direct advice and guidance from an emergency medicine specialist; and
a control unit configured to control operations and perform state monitoring of the patient information registration unit, the patient symptom input unit, the data security and personal information management unit, the emergency treatment guideline provision unit, the vehicle and emergency medical device interworking unit, and the remote medical support unit, to interwork with an existing medical information platform so as to utilize personal information of the patient, and to share information with the medical institution when necessary in the emergency situation to support rapid medical treatment.

2. The system of claim 1, further comprising:
an external medical data interworking unit for interworking with a national medical MyData platform so as to automatically retrieve and utilize medical records, underlying conditions, and allergy information, and the like of the patient.

3. The system of claim 1, wherein the essential information received by the patient information registration unit comprises at least one of a name, an age, a sex, contact information, a height, a weight, an underlying condition, medication information, and surgical history of the patient.

4. The system of claim 1, wherein the patient symptom input unit receives current symptoms of the patient in at least one form selected from the group consisting of text, an image, audio, a video, and biometric data.

5. The system of claim 1, wherein the emergency treatment guideline provision unit provides the appropriate emergency treatment method for the patient using an emergency treatment guideline generated on the basis of emergency medicine textbooks, latest papers and guidelines, and clinical experience of medical personnel, or a pre-trained artificial intelligence (AI)-based emergency treatment model.

6. The system of claim 1, wherein the vehicle and emergency medical device interworking unit monitors, in real time, changes in a condition of the patient using an in-vehicle sensor, and automatically contacts an emergency medical service center when necessary.

7. The system of claim 1, wherein, in a case of a non-autonomous vehicle, the vehicle and emergency medical device interworking unit provides optimal route guidance and emergency treatment guidance to a driver via real-time voice guidance and visual alerts.

8. The system of claim 1, wherein the vehicle and emergency medical device interworking unit is configured to interwork with an emergency medical device provided within the vehicle, including an automated external defibrillator (AED), an oxygen supply device, and a heart rate monitor, to monitor a biometric signal of the patient in real time and to automatically operate the emergency medical device when necessary.

9. The system of claim 1, wherein the remote medical support unit supports receipt of the advice and guidance via at least one of a video call, a voice call, and chat.

10. The system of claim 1, wherein the control unit interworks with the existing medical information platform to utilize the personal information of the patient, and the personal information of the patient comprises at least one selected from the group consisting of medical records, underlying conditions, and allergy information of the patient.

11. An emergency medical method for a vehicle for guiding laypersons in performing emergency patient classification, treatment, and transport to hospital, the method comprising:
a) receiving, by a patient information registration unit, essential information of a patient from the patient or an emergency responder, prior to boarding the vehicle or within the vehicle after boarding;
b) receiving, by a patient symptom input unit, current symptoms of the patient in various forms from the patient or the emergency responder;
c) providing, by an emergency treatment guideline provision unit, an appropriate emergency treatment method for the patient on the basis of the collected information;
d) interworking, by a vehicle and emergency medical device interworking unit, with sensor data and a control system of an autonomous vehicle, and supporting the vehicle to automatically travel to a nearest medical institution or stop at a safe location when an emergency situation occurs;
e) supporting, by a remote medical support unit, real-time communication with remote medical personnel within the vehicle so that the patient or the emergency responder can receive direct advice and guidance from an emergency medicine specialist;
f) interworking, by a control unit, with an existing medical information platform so as to utilize personal information of the patient; and
g) sharing, by the control unit, information with the medical institution when necessary in the emergency situation to support rapid medical treatment.

12. The method of claim 11, wherein, in step (a), the essential information received by the patient information registration unit comprises at least of a name, an age, a sex, contact information, a height, a weight, an underlying condition, medication information, and surgical history of the patient.

13. The method of claim 11, wherein, in step (b), the patient symptom input unit receives current symptoms of the patient in at least one form selected from the group consisting of text, an image, audio, a video, and biometric data.

14. The method of claim 11, wherein, in step (c), the emergency treatment guideline provision unit provides an appropriate emergency treatment method for the patient using an emergency treatment guideline generated on the basis of emergency medicine textbooks, latest papers, guidelines, and clinical experience of medical personnel, or a pre-trained artificial intelligence (AI)-based emergency treatment model.

15. The method of claim 11, wherein, in step (d), the vehicle and emergency medical device interworking unit monitors, in real time, changes in a condition of the patient using an in-vehicle sensor, and automatically contacts an emergency medical service center when necessary.

16. The method of claim 11, wherein, in step (d), in a case of a non-autonomous vehicle, the vehicle and emergency medical device interworking unit provides optimal route guidance and emergency treatment guidance to a driver via real-time voice guidance and visual alerts.

17. The method of claim 11, wherein, in step (d), the vehicle and emergency medical device interworking unit is configured to interwork with an emergency medical device provided within the vehicle, including an automated external defibrillator (AED), an oxygen supply device, and a heart rate monitor, to monitor a biometric signal of the patient in real time and to automatically operate the emergency medical device when necessary.

18. The method of claim 11, wherein, in step (e), the remote medical support unit supports receipt of the advice and guidance via at least one of a video call, a voice call, and chat.

19. The method of claim 11, wherein, in step (f), the personal information of the patient comprises at least one selected from the group consisting of medical records, underlying conditions, and allergy information of the patient.
